Europäisches Patentamt

(19) European Patent Office

Office européen des brevets

(11) Numéro de publication: **0 131 477**
**B1**

---

(12) **FASCICULE DE BREVET EUROPEEN**

---

(45) Date de publication du fascicule du brevet:
**24.06.87**

(21) Numéro de dépôt: **84400997.7**

(22) Date de dépôt: **16.05.84**

(51) Int. Cl.⁴: **A 61 K 37/02, C 07 K 5/06**

---

(54) Utilisation d'un sel de l'acide N-acetyl (alpha, beta) aspartylglutamique pour la préparation d'un médicament à activité anti-allergique pour administration locale.

---

(30) Priorité: **24.05.83 FR 8308495**

(43) Date de publication de la demande:
**16.01.85 Bulletin 85/3**

(45) Mention de la délivrance du brevet:
**24.06.87 Bulletin 87/26**

(84) Etats contractants désignés:
**AT BE CH DE GB IT LI NL**

(56) Documents cités:
**WO - A - 83/00146**
**FR - A - 2 257 270**

**EXPERIENTIA, volume XXI, no. 12, 1965, Birkhäuser Verlag (Basel, CH) E. MARCHETTI et al.: "On the structure of the natural dipeptide N-acetyl-aspartyl-glutamic acid (NAAGA)", pages 687-688**

(73) Titulaire: **THEA (Thérapeutique et Applications) S.A., 36 bis rue des Courtiaux, F-63000 Clermont-Ferrand (FR)**

(72) Inventeur: **Chibret, Henri, 42 Avenue Julien, F-63000 Clermont-Ferrand (FR)**

(74) Mandataire: **Thibon-Littaye, Annick et al, Cabinet A. THIBON-LITTAYE 11 rue de l'Etang, F-78160 Marly-le-Roi (FR)**

---

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente demande concerne l'utilisation d'un sel de l'acide N-acétyl-($\alpha$, $\beta$)-aspartylglutamique pour la préparation d'un médicament à activité anti-allergique pour administration locale, et qui permet donc, sous cette forme, de traiter diverses maladies, comme la conjonctivite, la rhinite et l'asthme bronchique, lorsqu'elles ont une origine allergique.

Les médicaments anti-allergiques de l'art antérieur peuvent être classés en trois catégories:
- les corticostéroïdes qui agissent sur les facteurs cellulaires de l'inflammation,
- les anti-dégranulants mastocytaires,
- les anti-histaminiques.

L'invention permet de proposer un médicament antidégranulant mastocytaire au moins équivalent au cromoglycate de sodium, considéré comme le médicament type de cette catégorie, mais présentant, en outre, une activité anti-complément qui s'est révélée fort utile, alors que personne ne s'était spécialement intéressé jusqu'à ce jour à cette propriété.

Le sel de l'acide N-acétyl-($\alpha$, $\beta$)-aspartylglutamique pour la préparation d'un médicament à activité anti-allergique pour administration locale se présente avantageusement alors en solution aqueuse isotonique, à une concentration comprise entre 1 et 6% et, de préférence, entre 2 et 4%, exprimée en masse d'acide pour 100 ml de solution.

L'acide N-acétyl-($\alpha$, $\beta$)-aspartylglutamique (NAAGA) est un composé déjà connu, ainsi d'ailleurs que divers de ses sels. Certaines propriétés pharmacologiques de ces composés sont également connues. Mais, jusqu'à présent, on n'avait envisagé de leur trouver des applications médicales qu'en tirant partie de leur activité pharmacologique sur le système nerveux central.

Ainsi, la demande de brevet français publiée sous le numéro 2 257 270 préconise l'emploi de sels de l'acide N-acétyl ($\alpha$, $\beta$)-aspartyl glutamique dans le traitement de la fatigue cérébrale, des états de choc, des états asthéniques. Et il est important de souligner que pour un tel emploi, les sels de l'acide N-acétyl ($\alpha$, $\beta$)-aspartyl glutamique sont incorporés dans des médicaments à administrer par voie générale, orale ou parentérale se présentant sous forme de comprimés, de gélules, de compositions buvables ou de solutés injectables.

A la base de la présente invention, on a constaté que le NAAGA et ses sels possèdent des propriétés d'anti-dégranulants mastocytaires qui n'avaient jamais été envisagées auparavant. Or on sait que la dégranulation des mastocytes et la libération des médiateurs de l'inflammation (histamine, prostaglandines et leucotriènes), qui en résulte, sont capitales dans la production des symptômes de l'allergie. De plus, on a découvert que ces mêmes composés présentent une activité anticomplément, ce qui est particulièrement intéressant puisqu'il a été montré que le système du complément joue un rôle important dans les mécanismes de l'inflammation, par l'intermédiaire de ses fractions anaphylatoxiques et chimiotactiques, à activité vasodilatatrice, d'augmentation de la perméabilité capillaire (fractions C3a, C5a, C4a) et de migration des cellules inflammatoires. Enfin, il est apparu que ces deux activités antagonistes vis-à-vis de facteurs importants de l'inflammation allergique, se trouvaient réunies dans l'utilisation selon l'invention, pour les concentrations indiquées de NAAGA, présent sous forme salifiée en solution isotonique, ce qui en fait un médicament intéressant, et original quant à son mode d'action, pour le traitement de l'allergie. L'utilisation thérapeutique en a été rendue possible par le fait que le médicament est d'une inocuité parfaite.

Il est important aussi de souligner que, d'après les essais effectués, les médicaments connus à base du même acide ou de ses sels, présentés à des concentrations adaptées pour une administration par voie générale, ne sont pas efficaces dans le traitement des maladies allergiques. Il est donc pleinement justifié pour la demanderesse, de revendiquer protection pour l'utilisation de ces sels sous des formes pharmaceutiques destinées à une application locale, sur les muqueuses, oculaires, nasales et bronchiques en particulier, permettant de mettre à profit l'activité thérapeutique anti-allergique nouvellement découverte de composés en eux-mêmes connus, étant donné que cette activité thérapeutique ne pouvait pas se manifester avec les formes pharmaceutiques antérieures.

Le médicament préparé selon l'invention est utile pour produire l'effet anti-allergique, notamment:
- au niveau de l'oeil dans le traitement des conjonctivites allergiques,
- au niveau du nez dans le traitement des rhinites allergiques,
- au niveau des bronches, dans le traitement de l'asthme d'origine allergique.

En fonction de sa destination, ce médicament sera conditionné sous la forme de solution nasale, de collyre, ou de solution pour aérosol bronchique, contenant des excipients classiques et des conservateurs suivant les exigences habituellement formulées pour ce genre de produits.

L'acide N-acétyl-($\alpha$, $\beta$)-aspartylglutamique et ses sels sont préparés selon des méthodes en elles-mêmes connues, telles que celles qui ont été décrites dans la demande de brevet français antérieur déjà citée (N° 2 257 270). Ils sont faciles à mettre en solution dans l'eau à la concentration utile pour l'utilisation dans la préparation de médicaments anti-allergiques selon l'invention. Dans ceux-ci, le NAAGA est avantageusement sous forme de ses sels de métaux alcalins ou alcalino-terreux, tels que les sels de sodium, de calcium, de magnésium, de ses sels d'amino-alcools, tels que les sels formés avec le diméthylaminoéthanol ou le diéthylaminoéthanol, de ses sels d'amino acides basiques tels que la lysine. D'une manière générale, il s'agit de tous sels pharmaceutiquement acceptables, formés avec des bases minérales ou organiques, qu'ils soient seuls ou en mélange. Ils peuvent éventuellement être utilisés dans des formes hydratées.

Dans une forme de réalisation préférée de l'utilisation des médicaments préparés présentent les particularités suivantes:

1) La solution est isotonique, avec une osmolarité comprise entre 250 et 350 m Osm, et de préférence voisine de 300 m Osm, que l'on peut ajuster par addition de chlorure de sodium, Cl Na, notamment.

2) Son pH est compris entre 6,5 et 7,5, et de préférence de l'ordre de 7,0 ± 0,3; il peut être ajusté avec Cl H ou Na OH.

3) La solution est stérile, par exemple telle qu'elle soit conforme à l'«essai de stérilité par filtration su sur membrane» de la Pharmacopée Française X Edition-V.2.I.I.

4) La solution contient un conservateur afin de garantir la stérilité de la solution pendant la durée de son utilisation (15 jours minimum après ouverture du flacon stérile). Ce conservateur est un conservateur choisi parmi ceux qui sont classiquement utilisés dans les solutions pour application locale (le chlorure de benzalkonium par exemple), ou l'un des conservateurs préconisés par la Pharmacopée Française, tels que l'acide p-hydroxy-benzoïque ou ses esters, l'alcool benzylique, le thiomersal, le chlorobutanol.

L'invention sera maintenant exposée plus en détail dans les exemples ci-après.

Exemple I
Solutions nasales

On utilise du NAAGA préparé selon l'exemple 7 de la demande de brevet antérieure déjà citée, ou du sel de magnésium hydraté obtenu selon son exemple 6, pour préparer des solutions nasales selon l'invention répondant aux formules suivantes:

Exemple I.1
- Sel de magnésium du NAAGA     3 g
  (correspondant à 2 g de
NAAGA acide)
- Chlorure de benzalkonium     0,01 g
- Chlorure de sodium     0,7 g
- Acide chlorhydrique     q.s.p. pH 6,8
- Eau purifiée     100 ml
L'osmolarité est de 300 m Osm.

Exemple I.2
- Sel de magnésium du NAAGA     6 g
  (correspondant à 4 g de
NAAGA acide)
- Chlorure de benzalkonium     0,01 g
- Chlorure de sodium     0,2 g
- Acide chlorhydrique 0,1 N     q.s.p. pH 6,8
- Eau purifiée     100 ml
L'osmolarité est de 300 m Osm.

La posologie sera de 1 à 2 pulvérisations dans chaque narine 3 à 5 fois par jour.

Exemple II
Collyres

On utilise de manière analogue des collyres selon l'invention répondant aux formules suivantes:

Exemple II.1
- Sel de magnésium du NAAGA     3 g
  (correspondant à 2 g de
NAAGA acide)
- Chlorure de benzalkonium     0,01 g
- Chlorure de sodium     0,7 g
- Acide chlorhydrique     q.s.p. pH 7,3
- Eau purifiée     100 ml

Exemple II.2
- NAAGA     4 g
- Chlorure de benzalkonium     0,01 g
- Chlorure de sodium     0,2 g
- Soude Na OH 0,1 N     q.s.p. pH 7,3
  (neutralise l'acide NAAGA)
- Eau purifiée     100 ml

Exemples II.3 et II.4

La composition dans les exemples est la même que dans l'exemple II.1, sauf que le NAAGA est spécifiquement soit en forme α (exemple II.3), soit en forme β (exemple II.4). Les formes α et β peuvent être obtenues isolément, par exemple en appliquant la méthode de préparation qui est décrite dans le brevet français 1 477 573, alors que la méthode de préparation utilisée dans les autres exemples conduit normalement à un mélange des formes α et β.

La posologie sera avantageusement de 1 goutte dans chaque oeil, 3 à 5 fois par jour, pour chacune de ces compositions.

Exemple III
Solution pour aérosol bronchique

Pour emploi en aérosol bronchique, on utilise la composition suivante:

- Sel de magnésium du NAAGA     3 g
  (correspondant à 2 g de
NAAGA acide)
- Chlorure de benzalkonium     0,01 g
- Chlorure de sodium     0,7 g
- Acide chlorhydrique 0,1 N     q.s.p; pH 6,8
- Eau distillée     100 ml

La posologie pourra être de 30 à 50 ml par jour, en nébulisation.

Exemple IV

Essais de tolérance aigüe des médicaments selon l'exemple I sur le mouvement ciliaire.

Introduction

Ces essais sont de caractère préliminaire pour tout produit destiné à être utilisé localement par voie nasale, soit sous forme d'instillations, soit en pulvérisations ou en aérosols. En effet, quel que soit l'intérêt thérapeutique d'un produit, il importe qu'il n'altère pas le mouvement ciliaire, élément moteur de la première «ligne de défense» des voies aériennes supérieures que constitue le «système muco-ciliaire», dont le rôle est d'épurer l'air que nous respirons, de nombreux polluants particulaires tels que poussières, allergènes divers, germes microbiens, qu'il contient en suspension.

Matériels et méthodes

La technique utilisée a été décrite sous le nom de microphoto-oscillographie. (L.C. CHEVANCE, J.F. LENNON: Etude des rythmes du battement ciliaire (Acta Otolaryng., 1970–70, 16–28)

On a utilisé pour cette étude des cobayes ou des lapins, indemnes de tout signe clinique d'infection des voies aériennes. Les animaux sont sacrifiés, puis on dissèque le septum nasal ou la trachée. Ces tissus sont placés dans du liquide de survie PBS et l'on prélève par grattage l'épithélium superficiel, sur une surface d'environ 1 mm². Ce prélèvement, qui contient de nombreuses cellules ciliées en mouvement, est placé dans une chambre d'observation, sous un microscope spécial, équipé d'un système microphotoélectrique permettant l'enregistrement des battements ciliaires en diascopie.

La mesure consiste à enregistrer les fluctuations de la lumière provoquées par l'image grossie 500 fois d'un cil en mouvement devant l'ouverture d'un diaphragme de dimensions adéquates. Après amplification, les fluctuations lumineuses sont enregistrées graphiquement.

Les altérations du mouvement ciliaire se traduisent par une diminution de la fréquence des battements ciliaires. Elles dépendent, d'une part de la toxicité cellulaire propre des substances mises en contact avec les cellules ciliées et, d'autre part, de deux facteurs physicochimiques d'importance capitale dans la physiologie du battement ciliaire: le pH et l'osmolarité des solutions.

Résultats

Les résultats obtenus figurent dans le tableau 1, pour les compositions des exemples I.1 (NAAGA 2%, sel Mg) et I.2 (NAAGA 4%, sel Mg):

Les experts toxicologues proposent un critère basé sur des données physiologiques pour évaluer la toxicité cellulaire d'une préparation destinée à l'administration par voie nasale, à savoir que: «peut être admise toute solution (ou suspension) qui ne diminue pas de plus de 50% le rythme initial du battement ciliaire après vingt minutes de contact». Toutes les valeurs obtenues avec les médicaments selon les exemples I.1 et I.2, après des temps de contacts qui dépassent 20 minutes, se situent très largement dans les limites de tolérance précédemment définies.

Des résultats analogues sont obtenus si l'on remplace le sel de magnésium du NAAGA par le sel de sodium préparé selon la demande de brevet déjà citée, ou par le sel de calcium, obtenu selon l'exemple 5 de la même demande de brevet.

Exemple V

Action du complément activé sur la muqueuse des voies aériennes supérieures.

Activité protectrice des médicaments de l'exemple I.

Introduction

Il a été démontré expérimentalement (M.ETEVANT, L.G. CHEVANCE, Ann. Immunol. (Inst. Pasteur) 1980–131D, 13–42) que lorsque la muqueuse ciliée des voies aériennes supérieures du lapin était mise en présence de complément activé par la voie classique ou alterne, il se produisait un arrêt très rapide des battements ciliaires et des lésions cytologiques très importantes au niveau des cellules épithéliales.

Cette destruction, provoquée par l'organisation des dernières séquences $C_5$ à $C_9$ du complément qui sont cytolytiques, a pu être observée sur des fragments de muqueuse nasale humaine.

D'un point de vue physiopathologique, ces lésions de la muqueuse respiratoire dues au complément jouent un rôle important, en altérant le mouvement ciliaire et en favorisant la pénétration et la persistance de particules allergéniques dans la muqueuse, amplifiant ainsi les réponses inflammatoires locales et, comme il a été indiqué précédemment, l'activation du complément libère les médiateurs impliqués dans les symtômes de l'allergie.

Nous avons étudié l'activité anti-complément de la composition selon les exemples I.1 et I.2 par l'enregistrement du mouvement ciliaire et au moyen de la microscopie électronique.

Matériels et méthodes

L'animal d'expérience est le lapin. Près le sacrifice de l'animal, la trachée est disséquée, puis découpée de façon à obtenir plusieurs fragments égaux de longueur appropriée (3 anneaux trachéaux environ). Ces anneaux sont ouverts puis répartis de la façon suivante:

– l'un des anneaux, servant de témoin, est placé dans du liquide de survie de Hanks;
– chacun des autres anneaux est placé dans 10 ml de chacune des solutions à tester, respectivement celle de l'exemple I.1 et celle de l'exemple I.2.

Après 15 mn d'incubation à 37 °C, on effectue une mesure de fréquence du battement ciliaire, puis on immerge les préparations dans 1 ml de sérum sanguin autologue.

On ajoute alors aux différentes préparations, du sulfate de dextrane (activateur du complément) à la concentration de 5 mg/ml, et l'on incube pendant 20 mn. A l'issue de ce temps d'incubation, on effectue une nouvelle mesure de la fréquence du battement ciliaire.

Dans un certain nombre d'expériences, les données micro-photo-oscillographiques ont été complétées par une étude cytologique, en microscopie électronique, des fragments trachéaux traités et des fragments témoins.

Résultats

Les résultats des différents essais effectués figurent dans le tableau 2. Ils mettent en évidence chez les préparations témoins, un arrêt complet du mouvement ciliaire après 20 mn de contact avec le complément activé.

Il convient de souligner que, dans les mêmes conditions expérimentales, mais lorsque le sérum n'est pas activé, on observe des battements ciliaires pendant plusieurs heures.

Si avant d'incuber la muqueuse dans le sérum activé, on l'incube durant 20 minutes dans la composition selon les exemples I.1 et I.2, on constate qu'après contact avec le sérum activé le mouvement ciliaire continue de battre sur un rythme normal.

Conclusion

La composition selon les exemples I.1 et I.2 protège de façon très nette et reproductible, la muqueuse respiratoire contre les lésions cytologiques induites par l'activation du complément.

Des résultats analogues sont obtenus quand le sel de magnésium du NAAGA est remplacé par le sel de diméthylaminoéthanol ou de diéthylaminoéthanol, préparé selon l'exemple 11 ou 12 de la demande de brevet antérieure déjà citée, ou par le sel de lysine obtenu selon l'exemple 7 de cette demande antérieure.

Exemple VI

Activité des compositions selon l'exemple II dans la conjonctivité allergique du lapin.

Méthode

Le protocole expérimental a été mis au point d'après des travaux de OKADA et Coll. (OKADA-SHIMADA Invest. Ophtalmol Vis. Sci. 1980, 19, 2, 176–181).

Le modèle de conjonctivite allergique utilisé consiste à effectuer chez le lapin une immunisation conjonctivale par injection locale d'anticorps anti gamma-globulines bovines produits chez le lapin. Le déclenchement de la conjonctivite est ensuite obtenu par injection intraveineuse de gamma-globulines bovines. Les effets de l'inflammation d'origine allergique sont observés par l'augmentation du poids des conjonctives et l'accroissement de la perméabilité capillaire conjonctivale à la sérum-albumine de lapin marquée à l'iode 125, injectée par voie intraveineuse avant l'injection de l'antigène.

Deux heures après l'injection de gamma-globulines bovines, les lapins sont sacrifiés, puis les yeux sont prélevés et les conjonctives disséquées, pesées et soumises à un comptage de radioactivité.

Trois paramètres sont recueillis:
– le poids des conjonctives,
– l'index de perméabilité conjonctivohématique, évalué par le rapport de la radioactivité de 1 mg de conjonctive à celle de 1 ml de sang,
– la quantité de sérum-albumine marquée retrouvée dans la conjonctive.

Pour l'évaluation de l'effet protecteur des produits à l'étude, on constitue plusieurs lots de six lapins, qui reçoivent des installations de collyre aux temps suivants:

J-3: 6 instillations de 2 gouttes dans chaque oeil;

J-2: 6 instillations de 2 gouttes dans chaque oeil;

J-1: 6 instillations de 2 gouttes dans chaque oeil;

J-0: 3 instillations à −8 h, −4 h, −0,5 h; avant l'injection de sérum albumine marquée.

Un lot de six lapins normaux, non traités et non soumis à la provocation allergique, est également constitué (lot «blanc»), ainsi qu'un lot de six lapins témoins recevant des instillations de sérum physiologique avant le déclenchement de la conjonctivite («témoins conjonctivites»).

Résultats

Les résultats obtenus, concernant les trois paramètres de l'inflammation conjonctivale d'origine allergique étudiés, sont rapportés dans le tableau 3.

En conclusion de ces essais, il apparaît que les collyres selon l'invention de l'exemple II, possèdent une activité protectrice significative contre l'inflammation conjonctivale d'origine allergique chez le lapin. Leur activité apparaît supérieure à celle du Cromoglycate disodique en collyre à 2%, utilisé comme médicament de référence. Les résultats des essais font ressortir également l'importance que présente le choix de la concentration, puisque le collyre selon l'exemple II.1 dilué au tiers est à considérer comme inactif.

D'autre part, ces résultats mettent en évidence que, à concentration de NAAGA égale, les formes $\alpha$ et $\beta$ ainsi que leurs mélanges, ont une activité équivalente.

Exemple VII

Action anti-dégranulante mastocytaire du NAAGA sur la muqueuse nasale du cobaye.

Méthode

Dans son principe, le test repose sur la détermination en microscopie du nombre de mastocytes granulés par unité de surface dans la muqueuse nasale du cobaye et sur l'étude de l'administration d'une substance histamino-libératrice – le 48/80 – sur ce nombre de mastocytes. L'animal d'expérience choisi est le cobaye. Il reçoit 2 fois par jour, pendant 3 jours dans chaque narine, 0,1 ml du produit à l'étude, 15 mn avant l'instillation nasale de 0,1 ml de 48/80 en solution à 2%, les animaux sont sacrifiés 1 heure après la dernière instillation.

Après sacrifice, la muqueuse nasale est prélevée, puis fixée et colorée au bleu de toluidine pour permettre une coloration sélective des mastocytes granulés. Pour chaque animal, on compte en microscopie les mastocytes contenus dans 100 champs microscopiques.

Résultats et conclusion

Comme le montre le tableau 4, la protection contre la dégranulation mastocytaire conférée par le traitement préventif local par les produits de l'invention à base de 2% ou 4% de NAAGA est totale. A titre de comparaison, le cromoglycate disodique à 2% (solution commerciale) utilisé dans la même condition expérimentale que les solutions à base de NAAGA, présente un effet protecteur inférieur d'environ 40% à celui du NAAGA.

Le médicament selon l'exemple I.1 ou I.2 de l'invention, administré par instillations locales, possède donc une très intéressante activité antidégranulante mastocytaire au niveau de la muqueuse nasale du cobaye. Son effet est nettement supérieur à celui du médicament de référence, le cromoglycate de sodium.

Exemple VIII

Etude clinique de la composition selon l'exemple II.2 dans le traitement des conjonctivites allergiques.

L'essai a été réalisé en double aveugle. Il avait pour objectif de déterminer les effets de la composition selon l'exemple II.2, solution ophtalmique à 4%, sur les symptômes oculaires et les signes inflammatoires conjonctivaux chez des malades atteints de conjonctivite allergique chronique, comparativement au cromoglycate disodique en solution ophtalmique à 2% utilisé en thérapeutique.

Les résultats de cette étude comparative portent sur 33 malades, des deux sexes, qui ont été répartis au hasard en deux groupes:

– 17 ont reçu la composition selon l'exemple II.2 pendant 2 semaines à raison de 1 goutte dans chaque oeil à traiter, 3 à 5 fois par jour. Ces malades ont ensuite reçu le cromoglycate disodique en collyre à 2% (collyre du commerce) pendant deux semaines supplémentaires et à la même posologie,

– 16 malades ont reçu les mêmes collyres dans l'ordre inverse et pendant les mêmes durées.

Le tirage au sort a assuré une bonne homogénéité dans la répartition entre les deux groupes de traitement, tant en ce qui concerne les caractéristiques cliniques que la durée d'administration des collyres à l'essai et que l'utilisation de traitements concomitants.

Les résultats obtenus sont les suivants:
Première période de traitement (jugement du clinicien):

|  | Composition de l'exemple II.2 | Cromoglycate disodique |
|---|---|---|
| Bons résultats | 11 | 11 |
| Résultats nuls | 6 | 5 |

Comparaison des 2 collyres d'après les préférences exprimées par les malades.

| Préférence pour la composition selon II.2 | 15 |
|---|---|
| Préférence pour le cromoglycate | 8 |
| Pas de préférence exprimée | 9 |

La tolérance oculaire a été jugée bonne pour les deux collyres.

Conclusions

Cette étude clinique en double insu, qui visait à comparer l'efficacité du NAAGA et du cromoglycate disodique en solutions ophtalmiques, a permis de mettre en évidence une amélioration des symptômes oculaires chez plus de 65% des patients, de façon comparable pour les deux produits.

L'analyse des préférences exprimées par les malades est nettement en faveur de la composition de l'exemple II.2 (67% des préférences en sa faveur contre 33% en faveur du cromoglycate).

Exemple IX

Etude clinique de la composition selon l'exemple III.

Cette étude a porté sur 6 sujets souffrant d'asthme par allergie à la poussière de maison, à la farine ou au foin.

On a recherché, chez ces malades, l'effet protecteur de la solution pour aérosol bronchique à 2% selon l'exemple III.

Dix ml de cette composition ont été nébulisés pendant 10 minutes avant la réalisation d'un test de provocation par l'allergène responsable de l'asthme chez ces patients.

On a comparé les résultats des mesures de débit expiratoire en spirométrie (VEMS), ainsi que les signes cliniques d'allergie respiratoire après NAAGA + provocation allergénique, par rapport à une épreuve antérieure de provocation chez le mêmes sujets, mais sans administration préalable de NAAGA.

Le tableau 5 résume les résultats obtenus chez les différents malades.

On notera une protection de la réaction broncho-constrictrice immédiate et/ou retardée due à l'inhalation d'allergène, dans 5 cas sur 6 (N° 1, 2, 3. 4 et 6).

Tableau 1
Etude de la tolerance aigue sur le mouvement ciliaire

| Produit testé | Nombre d'expérience | pH | Osmolarité (m Osm) | Animal, Tissu | Température ( °C) | Avant (PBS) | Après 20 mn | Après 40 mn |
|---|---|---|---|---|---|---|---|---|
| | | | | | | Rythme du battement ciliaire (cycles/mn) | | |
| NAAGA 2% (Ex. I.1) | 2 | 6,8 | 338 | Lapin, Muqueuse nasale, Trachée | 37 / 21 | 680 / 320 | 420 / 260 | 380 / 260 |
| NAAGA 2% (Ex. I.1) | 3 | 6,9 | 348 | Cobaye, Trachée | 21 / 21 / 21 | 400 / 500 / 360 | 360 / 480 / 360 | 400 / 400 / 400 |
| NAAGA 4% (Ex. I.2) | 3 | 6,8 | 300 | Cobaye, Trachée | 21 / 21 / 21 | 320 / 340 / 360 | 220 / 280 / 440 | 200 / 280 / 440 |

Tableau 2
Effets protecteurs vis a vis de l'action du complement active

| Substances testées (Concentration) | Expérience | Etude des battements ciliaires (cycles/mn) | | Etude en microscopie électronique (SEM) Après activation du complément |
|---|---|---|---|---|
| | | Avant activation du complément | 200 mn après activation du complément | |
| Témoins | 1 | 240 | En voie d'arrêt | Destruction complète des cils. |
| Solvant de la | 2 | 360 | Arrêt | Destruction complète des cils. |
| composition | 3 | 420 | Arrêt | Destruction complète des cils. |
| ajusté à | 4 | 300 | Arrêt | Destruction complète des cils. |
| 300 m Osm, | 5 | 220 | Arrêt | Destruction complète des cils. |
| avec conservateur | 6 | 300 | Arrêt | Destruction complète des cils. |
| | 7 | 320 | Arrêt | Destruction complète des cils. |
| NAAGA 2% | 8 | 360 | 460 | Aspect normal de la ciliature. |
| (Ex. I.1) | 9 | 300 | 320 | Aspect normal de la ciliature. |
| | 10 | 440 | 480 | Aspect normal de la ciliature. |
| | 11 | 280 | 300 | Aspect normal de la ciliature. |
| | 12 | 300 | 280 | Petite zone lésée. Aspect normal de la ciliature sur la majeure partie de la muqueuse. |
| | 13 | 480 | 380 | Aspect normal de la ciliature. Présence toutefois d'une petite zone lésée. |
| | 14 | 280 | 300 | Aspect normal de la ciliature sauf en 2 endroits où des lésions sont visibles. |
| | 15 | 340 | 300 | Aspect normal de la ciliature. |
| NAAGA 4% | 16 | 400 | 380 | Aspect normal. |
| (Ex. I.2) | 17 | 360 | 420 | Ciliature largement préservée. |
| | 18 | 280 | 300 | Aspect normal sauf en un endroit où quelques lésions sont visibles |

Tableau 3
Essai de la conjonctive allergique

| Lots de lapins | Quantité de sérum albumine dans les conjonctives (en micro g) | Index conjonctivo-hématique (X 100) | Poids des conjonctives (en mg) |
|---|---|---|---|
| 1. «Blancs» (n = 12) (yeux normaux) | 18,4 ± 5 | 2,53 ± 0,69 | 261 ± 58 |
| 2. Témoins conjonctivites (n = 12) | 51,6 ± 25 | 7,5 ± 3 | 664 ± 152 |
| 3. Cromoglycate (n = 12) de Na 2% (collyre du commerce) | 34,5 ± 13,6 | 4,7 ± 1,8 | 411 ± 64 |
| 4. Sel de magnésium de NAAGA 1% * (n = 12) | 54 ± 26 | 6,88 ± 3,73 | 465 ± 105 |
| 5. Sel de magnésium de NAAGA 3% (n = 12) (exemple II.1) | 27,5 ± 9,3 | 3,75 ± 1,3 | 282 ± 93 |
| 6. Sel de magnésium de NAAGA 6% (n = 12) (exemple II.2) | 27,4 ± 6,5 | 3,74 ± 1,93 | 345 ± 59 |
| 7. Sel de magnésium de l'$\alpha$-NAAGA 3% (n = 12) | 28,2 ± 6,2 | 3,96 ± 1,2 | 324 ± 52 |
| 8. Sel de magnésium de L-$\beta$-NAAGA 3% (n = 12) | 27,9 ± 7,1 | 4,05 ± 1,7 | 311 ± 61 |

n = nombre d'yeux
* = préparé par dilution au 1/3 du collyre à 3% selon l'exemple II.1 avec du sérum physiologique.

Tableau 4
Degranulation des mastocytes de la muqueuse nasale
du cobaye par instillations de – 48/80 –

Effets protecteurs du NAAGA à 2% et 4%
et du cromoglycate disodique

| Substances testées | Nombre moyen de mastocytes granulés par champ microscopique |
|---|---|
| Témoins normaux | 7,49 |
| | Instillation de 48/80 |
| Témoins dégranulés | 2,42 |
| NAAGA à 2%. Ex. I.1. | 7,8 |
| NAAGA à 4%. Ex. I.2. | 7,0 |
| Cromoglycate disodique à 20 mg/ml (solution commerciale) | 4,6 |

Tableau 5
V.E.M.S.: Vitesse Expiratoire Maximale/s

Rhin = Rhinite, Sib = Sibilants

Etude clinique – asthme

| Malades N° | Allergène | Epreuve | % de variation des V.E.M.S. Après/Avant | | Clinique | | | |
|---|---|---|---|---|---|---|---|---|
| | | | Réaction immédiate | Réaction retardée | Réaction immédiate | | Réaction retardée | |
| N° 1 | Poussière | Témoin | | −19% | | | Rhin +++ | Sib ++ |
| REZ | de maison | Ex. III | Absente | − 9% | Absente | | Rhin++ | Sib 0 |
| N° 2 | Farine | Témoin | −15% | Absente | Rhin +++ | Sib ++ | Absente | |
| HAR | | Ex. II | 0% | | Rhin 0 | Sib 0 | | |
| N° 3 | Poussière | Témoin | −13% | −35% | Rhin + | Sib ++ | Rhin +++ | Sib +++ |
| REY | de maison | Ex. III | 0% | +23% | Rhin + | Sib ++ | Rhin 0 | Sib + |
| N° 4 | Poussière | Témoin | −10% | Absente | Rhin +++ | Sib ++ | Rhin ++ | Sib +++ |
| CHA | de maison | Ex. III | + 8% | | Rhin + | Sib 0 | Rhin 0 | Sib 0 |
| N° 5 | Poussière | Témoin | − 9% | Absente | Rhin +++ | Sib ++ | Absente | |
| GAR | de maison | Ex. III | + 7% | | Rhin + | Sib 0 | | |
| N° 6 | Foin | Témoin | −30% | −12% | Rhin ++ | Sib ++ | Rhin + | Sib + |
| OUH | | Ex. III | −34% | + 2% | Rhin + | Sib ++ | Rhin + | Sib + |

On relèvera en particulier que suivant le mode de préparation des composés qui est apparu préférable, les composés α et β sont présents en mélange dans le médicament. On peut toutefois aussi appliquer l'invention en utilisant des composés spécifiquement obtenus sous la forme α ou la forme β.

## Revendications

1. Utilisation d'un sel de l'acide N-acétyl-(α, β)-aspartylglutamique pour la préparation d'un médicament à activité anti-allergique pour administration locale.

2. Utilisation d'un sel de l'acide N-acétyl-(α, β)-aspartylglutamique selon la revendication 1, caractérisée en ce que ledit sel est choisi parmi les sels de sodium, calcium, magnésium, et les sels d'amino-alcools ou amino-acides basiques pharmaceutiquement acceptables, et présent en solution isotonique à une concentration comprise entre 1 et 6%, exprimée en poids d'acide par rapport au poids total de la solution.

3. Utilisation d'un sel de l'acide N-acétyl-(α, β)-aspartylglutamique selon la revendication 2, caractérisée en ce que ladite concentration est comprise entre 2 et 4%.

4. Utilisation d'un sel de l'acide N-acétyl-(α, β)-aspartylglutamique selon la revendication 2 ou 3, caractérisée en ce que la solution présente une osmolarité comprise entre 250 et 350 m Osm et un pH compris entre 6,5 et 7,5.

5. Utilisation d'un sel de l'acide N-acétyl-(α, β)-aspartylglutamique selon la revendication 4, caractérisée en ce que l'osmolarité est voisine de 300 m Osm et le pH est de l'ordre de 7 (± 0,3).

6. Utilisation d'un sel de l'acide N-acétyl-(α, β)-aspartylglutamique selon l'une quelconque des revendications 2 à 5, caractérisée en ce que la solution contient un conservateur propre à la maintenir stérile pendant au moins 15 jours, qui est l'acide p-hydroxy-benzoïque ou ses esters, l'alcool benzylique, le thiomersal ou le chlorobutanol.

7. Utilisation d'un sel de l'acide N-acétyl-(α, β)-aspartylglutamique selon l'une quelconque des revendications 1 à 6, caractérisée en ce que ledit sel est présenté sous forme de collyre, de gouttes nasales, ou d'aérosol bronchique.

8. Utilisation d'un sel de l'acide N-acétyl-(α, β)-aspartylglutamique selon l'une quelconque des revendications 1 à 7, caractérisée en ce que ledit sel renferme les formes α et β de l'acide N-acétyl-aspartyl-glutamique.

9. Utilisation d'un sel de l'acide N-acétyl-(α, β)-aspartylglutamique selon l'une quelconque des revendications 1 à 7, caractérisée en ce que ledit sel renferme spécifiquement la forme α ou la forme β dudit acide.

## Patentansprüche

1. Verwendung eines Salzes der N-Acetyl-(α/β)-aspartylglutaminsäure zur Herstellung eines Medikamentes mit einer anti-allergischen Aktivität bei lokaler Applikation.

2. Verwendung eines Salzes der N-Acetyl-(α/β)-aspartylglutaminsäure nach Anspruch 1, dadurch gekennzeichnet, dass das genannte Salz aus den Salzen des Natriums, Kalziums, Magnesiums und den Salzen von Amino-Alkoholen oder basischen Aminosäuren, die pharmazeutisch akzeptabel sind, ausgewählt wird und in einer isotonischen Lösung mit einer Konzentration zwischen 1 und 6%, ausgedrückt in Gewicht der Säure in Bezug auf das Gesamtgewicht der Lösung, gelöst ist.

3. Verwendung eines Salzes der N-Acetyl-(α/β)-aspartylglutaminsäure nach Anspruch 2, dadurch gekennzeichnet, dass die genannte Konzentration zwischen 2 und 4% liegt.

4. Verwendung eines Salzes der N-Acetyl-(α/β)-aspartylglutaminsäure nach Anspruch 2 oder 3, dadurch gekennzeichnet, dass die Lösung eine Osmolarität zwischen 250 und 350 mOsm und einen pH-Wert zwischen 6,5 und 7,5 besitzt.

5. Verwendung eines Salzes der N-Acetyl-(α/β)-aspartylglutaminsäure nach Anspruch 4, dadurch gekennzeichnet, dass die Osmolarität nahe bei 300 mOsm liegt und der pH-Wert einen Wert von 7 (± 0,3) aufweist.

6. Verwendung eines Salzes der N-Acetyl-(α/β)-aspartylglutaminsäure nach einem der Ansprüche 2 bis 5, dadurch gekennzeichnet, dass die Lösung ein Konservierungsmittel enthält, um sie wenigstens 15 Tage steril zu halten, das aus p-Hydroxy-benzoesäure oder ihren Estern, aus Benzylalkohol, aus Thiomersalzen oder aus Chlorbutanol besteht.

7. Verwendung eines Salzes der N-Acetyl-(α/β)-aspartylglutaminsäure nach einem der Ansprüche 1 bis 6, dadurch gekennzeichnet, dass das genannte Salz in Form von Augentropfen, Nasentropfen oder von Bronchialaerosol angeboten wird.

8. Verwendung eines Salzes der N-Acetyl-(α/β)-aspartylglutaminsäure nach einem der Ansprüche 1 bis 7, dadurch gekennzeichnet, dass das Salz die α und β-Formen der N-Acetylaspartylglutaminsäure umfasst.

9. Verwendung eines Salzes der N-Acetyl-(α/β)-aspartylglutaminsäure nach einem der Ansprüche 1 bis 7, dadurch gekennzeichnet, dass das Salz spezifisch die α-Form oder die β-Form der Säure umfasst.

## Claims

1. Utilization of a salt of N-acetyl-(α, β)-aspartylglutamic acid for the preparation of a drug having an anti-allergic activity for local administration.

2. Utilization of a salt of N-acetyl-(α, β)-aspartylglutamic acid in accordance with claim 1, characterized in that said salt is chosen from the salts of sodium, calcium, magnesium, and the pharmaceutically acceptable amino-alcohol salts or basic amino-acids, and present in isotonic solution at a concentration comprised between 1 and 6% expressed by weight of acid with respect to the total weight of the solution.

3. Utilization of a salt of N-acetyl-(α, β)-aspartylglutamic acid in accordance with claim 2, characterized in that said concentration is comprised between 2 and 4%.

4. Utilization of a salt of N-acetyl-($\alpha$, $\beta$)-aspartylglutamic acid in accordance with claim 2 or 3, characterized in that the solution has an osmolarity comprised between 250 and 350 m Osm and a pH comprised between 6,5 and 7,5.

5. Utilization of a salt of N-acetyl-($\alpha$, $\beta$)-aspartylglutamic acid in accordance with claim 4, characterized in that the osmolarity is in the vicinity of 300 m Osm and the pH is of the order of 7 ($\pm$ 0.3).

6. Utilization of a salt of N-acetyl-($\alpha$, $\beta$)-aspartylglutamic acid in accordance with any one of claims 2 to 5, characterized in that the solution contains a preservative which is intended to maintain it in the sterile state for at least 15 days and which is p-hydroxy-benzoic acid or its esters, benzyl alcohol, thiomersal or chlorobutanol.

7. Utilization of a salt of N-acetyl-($\alpha$, $\beta$)-aspartylglutamic acid in accordance with any one of claims 1 to 6, characterized in that said salt is presented in the form of eye lotion, nasal drops or bronchial aerosol.

8. Utilization of a salt of N-acetyl-($\alpha$, $\beta$)-aspartylglutamic acid in accordance with any one of claims 1 to 7, characterized in that said salt contains the $\alpha$ and $\beta$ forms of N-acetyl-aspartylglutamic acid.

9. Utilization of a salt of N-acetyl-($\alpha$, $\beta$)-aspartylglutamic acid in accordance with any one of claims 1 to 7, characterized in that said salt specifically contains the $\alpha$ form or the $\beta$ form of said acid.